# EUROPEAN PATENT APPLICATION

(11) **EP 4 474 808 A1**
(43) Date of publication of application: **11.12.2024**
(21) Application number: 23178096.6
(22) Date of filing: 07.06.2023
(51) Int. Cl.: G01N 27/327, G01N 33/483, G01N 33/487, G01N 33/543, G01N 33/94

(54) **A CAPACITIVE MICROELECTRODE DEVICE FOR SENSING A BIOLOGICAL ACTIVITY WITHIN A SAMPLE AND A METHOD OF USING SUCH A MICROELECTRODE DEVICE**

(71) Applicant: ETH Zurich, 8092 Zurich (CH)
(72) Inventor: WEAVER, Sean Michael, 8704 Herrliberg (CH); RENZ, Aline Fabianna, 1012 Lausanne (CH); VÖRÖS, Janos, 8050 Zürich (CH); NAKATSUKA, Nako, 8800 Thalwil (CH); JANG, Taekwang, 8057 Zürich (CH); CHOI, Woojun, 8006 Zürich (CH)
(74) Representative: Gygi, Andreas

(57) **Abstract**

A capacitive microelectrode device (1), preferably a microelectrode array, for sensing a biological activity within a sample (S), the microelectrode device (1) comprises an electrode assembly (11) with electrodes (11.1, 11.2; 11.r, 11.c) for contacting the sample (S), and an electronic equipment (12) which is connected to the electrodes (11.1, 11.2; 11.r, 11.c) of the electrode assembly (11). The electronic equipment (12) is configured to evaluate one or more capacitances (C; C0, C1, ...) between the electrodes (11.1, 11.2; 11.r, 11.c) for sensing the biological activity within the sample (S). The microelectrode device (1) enables that the one or more capacitances (C; C0, C1, ...) include one or more electrical double layer capacitances (C_EDL) of one or more electrical double layers which are formed when the electrodes (11.1, 11.2; 11.r, 11.c) are brought into contact with the sample (S).

## Description

### FIELD OF THE INVENTION

The present invention relates to a microelectrode device for sensing a biological activity within a sample and a method of using such a microelectrode device.

### BACKGROUND ART

In neuroscience, measuring biological activity of neurons is central. Biological activity can relate to electrical activity from neurons firing action potentials. Biological activity can relate to chemical activity from neurotransmitters being released.

Microelectrode arrays (MEA) enable the simultaneous recording of the electrical activity of neurons via multiple electrodes. Complementary metal-oxide semiconductor (CMOS) MEA systems can have integrated >10,000 on-chip electrodes using metal pads with a pitch of ^{~}10µm using active pixel sensors (APS) or switchmatrix (SM) or sub-array multiplexing (SAM) [1-4]. However, they suffer from extreme area and power constraints on the readout circuits [1,2] or limit the number of concurrently recorded channels [3,4]. Moreover, neurons must be cultured on top of CMOS MEAs, making it difficult to accommodate various experimental setups such as transparent or stretchable electrodes. The materials of the chip can also corrode during long-term exposure to liquids, incurring extra packaging complexity to protect the chip and to prevent the cells from being damaged by the materials released by the chip [6].
[1] X. Yuan et al., JSSC, 2021; [2] Y. Kato et al., VLSI, 2020; [3] C. M. Lopez et al., JSSC, 2018; [4] J.-H. Cha et al., ISSCC, 2022; [5] D. Tsai et al., Nat. Comm., 2017; [6] A. Hierlemann et al., IEDM, 2015

Aptamers are single-stranded oligonucleotides that are designed in vitro to bind specific analytes of interest. There are aptamers that undergo large conformational rearrangements of negatively charged phosphodiester backbones in the presence of specific analytes. Aptamers can have a high selectivity, as the structure switching only occurs in the presence of the specific analyte for which they have been designed (contrary to conventional bioreceptors such as antibodies that have challenges of cross-reactivity and promiscuity).

### DISCLOSURE OF THE INVENTION

There is a need for an improved microelectrode device for sensing biological activity within a sample. There may be a need for an improved method of using such a microelectrode device.

Such a need may be met with the subject-matter of the independent claims. Advantageous embodiments are defined in the dependent claims.

Ideas underlying embodiments of the present invention may be interpreted as being based, inter alia, on the following observations and recognitions.

An aspect of the invention relates to a microelectrode device for sensing a biological activity within a sample. The microelectrode device comprises an electrode assembly with electrodes for contacting the sample, and an electronic equipment which is connected to the electrodes of the electrode assembly. The electronic equipment is configured to evaluate one or more capacitances between the electrodes for sensing the biological activity within the sample. The microelectrode device enables that the one or more capacitances include one or more electrical double layer capacitances of one or more electrical double layers which are formed when the electrodes are brought into contact with the sample. Evaluating a capacitance between two electrodes can relate to measuring the capacitance, i.e. the mutual capacitance. Evaluating capacitances between electrodes can relate to detecting a change in a capacitance. The electrode assembly can be configured for contacting the sample and the electronic equipment can be configured to apply a bias, an AC (alternate current) potential, a current, etc. to the sample such that the electrical double layer capacitance can be modulated. By detecting a change in the electrical double layer capacitance, various biological activities can be sensed within the sample.

According to an embodiment of the invention, the biological activity relates to an electrical activity within the sample, and the electronic equipment is configured to evaluate the one or more capacitances for sensing the electrical activity. The electrical activity can relate to an electrochemical activity of ions being transported from the outside of a biological cell, such as a neuron, into the inside and/or from ions being transported from the inside of a biological cell, such as a neuron, into the outside. The electronic equipment can be configured to evaluate frequencies in a range of 100-5000 Hz or similar, which are typical for electrical activities.

According to an embodiment of the invention, the biological activity relates to a chemical activity within the sample, and the electrode assembly is functionalized, in particular with aptamers i(a), for modifying the electrical double layer capacitance depending on the chemical activity, and the electronic equipment is configured to evaluate the one or more capacitances for sensing the chemical activity. Aptamers can be designed to chemically bind to target molecules, such as neurotransmitters like serotonin or dopamine, wherein the aptamers undergo a conformational change, such as regards length, shape, charge distribution, etc., when binding to the target molecules, thereby modifying the electrical double layer and the electrical double layer capacitance. In particular, electrical activity and chemical activity can be sensed at the same time. The electronic equipment can be configured to evaluate frequencies of 1 Hz, 0.1 Hz or similar, which are typical for chemical activities.

According to an embodiment of the invention, the electrode assembly includes electrodes which are arranged in a form of an array. The array can be designed to sense an area of the sample, such as a rectangular or quadratic area, such as an area of 500 micrometer x 500 micrometer, 1 millimeter x 1 millimeter, etc.

According to an embodiment of the invention, the electrode assembly includes electrodes which form a cross-coupled capacitive microelectrode array, and the electronic equipment is configured to apply a code division multiplexing method for evaluating the one or more capacitances. A compact, flexibly configurable electrode assembly is enabled, while interconnects between the electrode assembly and the electronic equipment are minimized. For example, a methodology can be applied for creating an electrical double layer capacitance on a CMOS chip. For example, the electrode assembly does not have to be an external cross-coupled array, it can be any metal electrode or metal electrodes on-chip or off-chip. For example, a capacitance is applied to analog or digital conversion circuits to measure the electrical double layer capacitance for sensing a biological activity such as action potential or neural activity. For example, a capacitance to an analog or digital conversion circuit can convert an electrical double layer capacitance of an array of electrical double layer capacitances to an analog voltage, an analog current, an analog impedance and to digital values.

According to an embodiment of the invention, the electronic equipment is configured to enable sensing of the biological activity with a frequency of 2 kHz or more. Thus, the microelectrode device enables sensing biological activity in the form of an electrical activity and of a chemical activity.

According to an embodiment of the invention, the electrode assembly is configured to enable sensing of the biological activity with a pitch of 50 micrometers or less. Thus, the microelectrode device enables sensing biological activity with a high spatial resolution.

According to an embodiment of the invention, the electrode assembly has a transparent configuration, in particular enabling the use of standard microscopy equipment.

The invention further relates to a method of using a microelectrode device as described. The method comprises: providing the microelectrode device, providing a sample, bringing the sample in contact with the electrodes of the electrode assembly, and enabling forming within the sample of one or more electrical double layers having one or more electrical double layer capacitances at one or more surfaces of the electrode, operating the electronic equipment to evaluate the one or more capacitances for sensing the biological activity.

According to an embodiment of the invention, the biological activity relates to an electrical activity, and the electronic equipment is operated for sensing the electrical activity.

According to an embodiment of the invention, the biological activity relates to a chemical activity, and the method further comprises functionalizing the electrode assembly, in particular with aptamers, for modifying the electrical double layer capacitance depending on the chemical activity, and the electronic equipment is operated for sensing the chemical activity.

According to an embodiment of the invention, functionalizing the electrode assembly includes one or more of: an in-flow functionalization, an outside functionalization, a hybrid functionalization, and a microfluidics functionalization.

According to an embodiment of the invention, the sample includes one or more of electrogenic cells, chemogenic cells and cells that have ion channel activities.

According to an embodiment of the invention, the sample includes one or more of neurons, glia, muscle cells, and cardiomyocites.

According to an embodiment of the invention, the sample is provided in-vitro or in-vivo.

### BRIEF DESCRIPTION OF THE DRAWINGS

In the following, advantageous embodiments of the invention will be described with reference to the enclosed drawings. However, neither the drawings nor the description shall be interpreted as limiting the invention.
Fig. 1 schematically shows a microelectrode device according to an embodiment of the present invention.
Fig. 2 schematically shows a neuron and an electrical double layer having an electrical double layer capacitance.
Fig. 3a, 3b schematically show an electrode functionalized with aptamers and an electrical double layer having an electrical double layer capacitance.
Figs. 4a, 4a' schematically show an embodiment of a microelectrode device according to the present invention and a respective equivalent circuit.
Fig. 4b schematically shows a frequency response of an impedance with and without neural activity of the embodiment of the microelectrode device according to Fig. 4a.
Fig. 5a schematically shows an embodiment of a microelectrode device with improved sensor selectivity according to an embodiment of the present invention.
Fig. 5b shows an electrode assembly according to the embodiment of the microelectrode device shown in Fig. 5a.
Fig. 6 schematically shows an embodiment of a microelectrode device with a cross-coupled capacitive microelectrode according to an embodiment of the present invention.
Fig. 7 schematically shows a signal processing method which enables evaluating each capacitance of the microelectrode device according to Fig. 6.
Fig. 8a shows an embodiment of an electrode assembly of a microelectrode device according to the present invention.
Fig. 8b shows a SEM (scanning electron microscopy) image of a cutout of the electrode assembly according to Fig. 8a.
Figs. 8c, 8c', 8c" schematically show 3-dimensional structures of a cutout of the electrode assembly according to Figs. 8a, 8b.
Fig. 9a shows a fluorescent image of an electrode assembly of a microelectrode device according to the present invention having arranged a sample with neurons.
Fig. 9b shows for a particular location an evaluation of the capacitance of the electrode assembly according to Fig. 9a.
Fig. 10 shows a sequence of activity maps of an electrode assembly of a microelectrode device according to the present invention.
Fig. 11a, 11b, 11c show an activity at adjacent locations of the electrode assembly according to Fig. 10.
Fig. 12 shows schematically possible method steps of a method of using a microelectrode device according to the present invention.

The figures are only schematic and not to scale. Same reference signs refer to same or similar features.

### MODE(S) FOR CARRYING OUT THE INVENTION

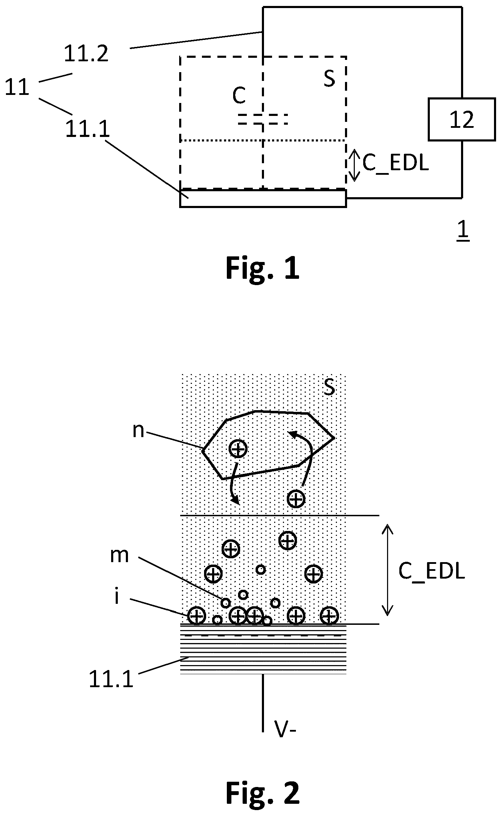

Fig. 1 schematically shows a microelectrode device 1 according to an embodiment of the present invention for sensing a biological activity within a sample S. For example, the sample S can relate to in-vitro or in-vivo tissue with neurons. For example, the biological activity is a physiological activity, e.g. signaling, metabolism, etc.

The microelectrode device 1 comprises an electrode assembly 11 and electronic equipment 12. The electrode assembly includes electrodes 11.1, 11.2 which are configured for contacting the sample S. According to the embodiment illustrated in Fig. 1, the electrode assembly 11 includes a first and a second electrode 11.1, 11.2. The electronic equipment 12 is connected to the electrodes 11.1, 11.2 of the electrode assembly 11, for example via electrically conductive wires. The electronic equipment 12 is configured to evaluate a capacitance C between the electrodes 11.1, 11.2 for sensing the biological activity within the sample. The microelectrode device 1 enables that the capacitance C includes an electrical double layer capacitance C_EDL of an electrical double layer which is formed when the electrodes 11.1, 11.2 are brought into contact with the sample S. According to the embodiment illustrated in Fig. 1, the electrical double layer having electrical double layer capacitance C_EDL is formed at a surface of the first electrode 11.1. For example, sensing a biological activity can relate to a single electrical double layer capacitance.

Fig. 2 schematically shows an embodiment of a sample S which is in contact with the first electrode 11.1. The sample S includes ions i, molecules m and a neuron n. For example, the sample S relates to an in-vitro or in-vivo sample of tissue with neurons. As illustrated in Fig. 2, the first electrode 11.1 is negatively charged, which effects that positively charged ions are accumulated on the surface of the first electrode 11.1. This results in the formation of an electrical double layer having an electrical double layer capacitance C_EDL. As illustrated in Fig. 2, when the neuron is activated, an electrical activity occurs because of the electrochemical activity of ions i which are transported from the outside of the neuron n to the inside and/or from the inside of the neuron n to the outside. This transfer of ions i also effects a change in the ion concentration within the electrical double layer, which results in a change of the electrical double layer capacitance C_EDL. Thus, detecting a change in the capacitance C between the electrodes 11.1, 11.2, which includes the electrical double layer capacitance C_EDL, sensing a biological activity in the form of an electrical activity is enabled.

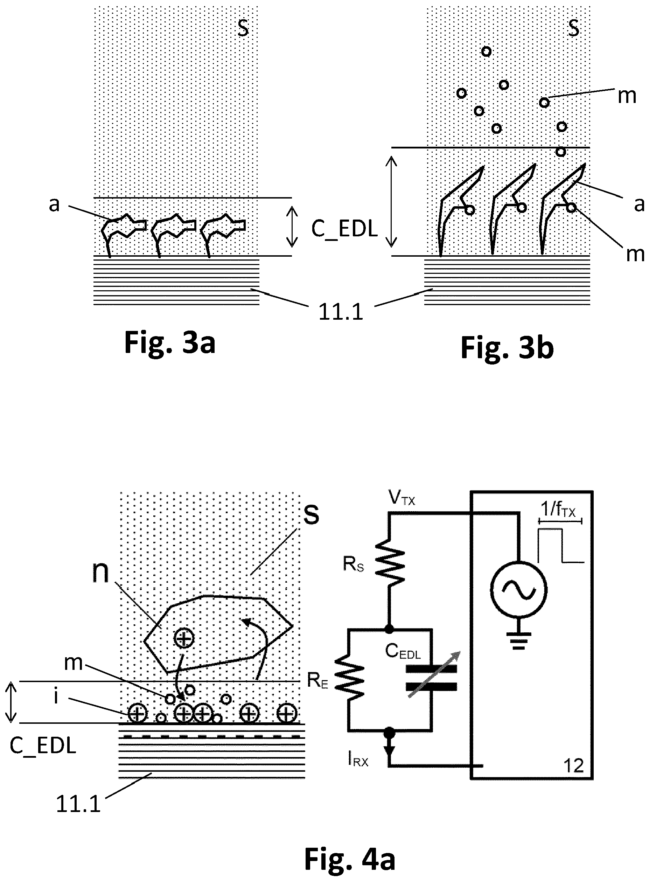

Fig. 3a, 3b schematically show a sample S which is in contact with the first electrode 11.1. As illustrated in Fig. 3a, the surface of the first electrode 11.1 is functionalized with aptamers a, wherein an electrical double layer having electrical double layer capacitance C_EDL is formed. The aptamers a are designed to chemically bind to target molecules m, for example to neurotransmitters such as serotonin or dopamine. As illustrated in Fig. 3b, the aptamers a undergo a conformational change (length, shape, charge distribution, etc.) when binding to the target molecules m, such as serotonin or dopamine released by neurons, thereby modifying the electrical double layer, such as its length, ion concentration, etc., and the electrical double layer capacitance C_EDL. Thus, functionalizing the first electrode 11.1 with aptamers a and detecting a change of the electrical double layer capacitance C_EDL enables sensing a biological activity in the form of a chemical activity.

In neuroscience, electrical activities have a frequency range of, for example, between 100 Hz - 5 kHz, while chemical activities have a frequency of for example below 1 Hz, such as 0.1 Hz. Accordingly, by spectral separation, electrical activities within the sample S can be separated from chemical activities. For example, chemical activities can relate to an accumulation of small, fast chemical events, wherein the accumulation results in a change of the electrical double layer capacitance in a frequency range of below 1 Hz, such as 0.1 Hz.

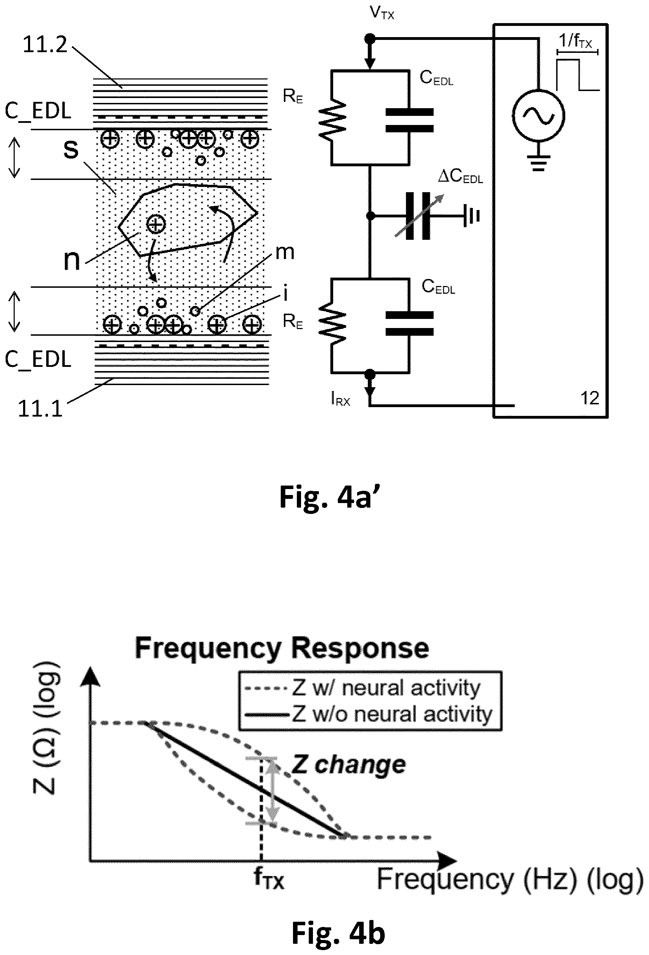

Fig. 4a schematically shows a microelectrode device 1 according to an embodiment of the present invention. The electrode assembly includes an electrode 11.1 which is configured for contacting a sample S. The electronic equipment 12 is connected to the sample S and includes a signal generator for transmitting a transmit signal V_TX to the sample S. The electronic equipment 12 is connected to the electrode 11.1 for receiving a receive signal I_RX (in the form of a current signal) from the electrode 11.1, which can be converted into a voltage signal V_TX within the electronic equipment 12 . Fig. 4a illustrates the equivalent circuit between the transmit signal V_TX and the receive signal I_RX when the sample S is brought into contact with the electrode 11.1. The equivalent circuit includes a fluid resistor R_S which is in serial connection with an electric double layer capacitor C_EDL, which is in parallel connection with an electrode resistor R_E. The surface charge of the electrode 11.1 in solution is balanced by oppositely charged counter ions accumulating at the surface, forming an electric double layer having electric double layer capacitance C_EDL. The counter ions in solution reside at the surface, separated by a distance on the order of 1nm for physiological conditions, resulting in a large capacitance on the order of for example ^{~}170 pF for 100 square micrometers. As a result of biological activity, the neuron can depolarize during an action potential, wherein the electrochemical potential of the ions on the surface changes, causing a corresponding change in the electric double layer capacitance C_EDL.

Fig. 4a' schematically shows a microelectrode device 1 according to an embodiment of the present invention. Contrary to Fig. 4a, in Fig. 4a' a first and a second electrode 11.1, 11.2 are shown. In the equivalent circuit, each electrode corresponds to an electrode resistor R_E connected in parallel with an electric double layer capacitor C_EDL, wherein between them, a delta electrical double layer capacitance ΔC_EDL is connected to ground, which corresponds to a change of the electrical double layer capacitance because of an electrical and/or a chemical activity within the sample S.

Fig. 4b schematically shows the frequency response of the impedance Z with neural activity and without neural activity of the embodiment of the microelectrode device 1 according to Fig. 4a.

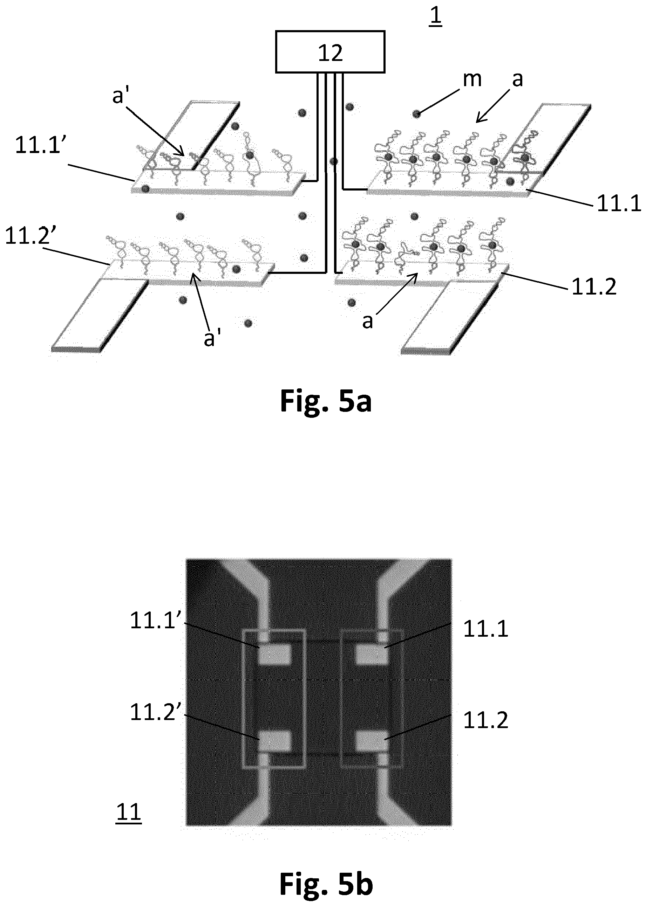

Fig. 5a schematically shows a microelectrode device 1 according to an embodiment of the present invention. The electrode assembly includes a first and second sensing electrode 11.1, 11.2, and a first and second reference electrode 11.1', 11.2'. The first and second sensing electrodes 11.1, 11.2 are functionalized with an aptamer a designed for molecules m, such as a neurotransmitter. The first and second reference electrodes 11.1', 11.2' are functionalized with a scrambled aptamer a' which is designed to not interact with the molecules m. The scrambled aptamer a' has the same chemical signature as the aptamer a (charge, mass, functional groups). The sensing electrodes 11.1, 11.2 and the reference electrodes 11.1', 11.2' are connected to the electronic equipment 12. The electronic equipment 12 is configured to evaluate the capacitance between the sensing electrodes 11.1, 11.2 and the capacitance between the reference electrodes 11.1', 11.2'. The capacitance between the reference electrodes 11.1', 11.2' is C' and does not change due to the presence of the molecules m. The capacitance between the sensing electrodes 11.1, 11.2 is C and changes upon the presence of the molecules m. The electronic equipment 12 can be configured to subtract the capacitance C' between the reference electrodes 11.1', 11.2' from the capacitance C between the sensing electrodes 11.1, 11.2 in order to improve sensor selectivity.

Fig. 5b shows an electrode assembly 11 according to the embodiment of the microelectrode device 1 shown in Fig. 5a.

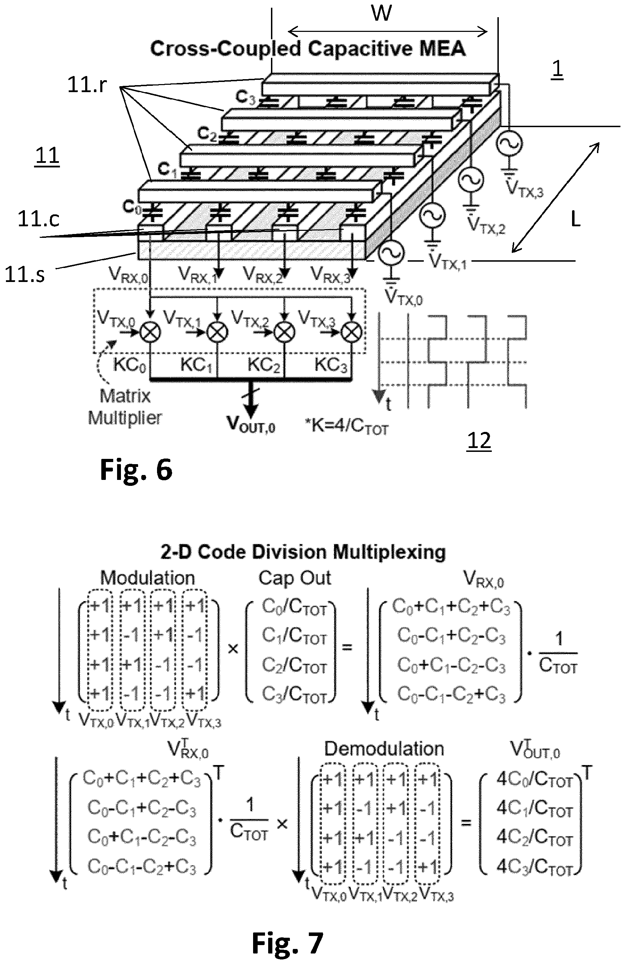

Fig. 6 schematically shows a microelectrode device 1 according to an embodiment of the present invention. The microelectrode device 1 includes an electrode assembly 11 and an electronic equipment 12. As illustrated in Fig. 6, the electrode assembly 11 includes electrodes 11.r, 11.c which form a cross-coupled capacitive microelectrode array. As illustrated in Fig. 6, a substrate 11.s such as a silicon substrate or similar having length L and width W supports a set of column electrodes 11.c which can have, e.g., the form of rectangular cuboids. In direction of the width W of the substrate 11.s, the column electrodes 11.c are separated from each other. In direction of the length L of the substrate 11.s, the column electrodes 11.c have length L. As illustrated in Fig. 6, a similar set of row electrodes 11.r is arranged in orthogonal direction above the column electrodes 11.c and separated from the column electrodes 11.c by an isolator (not shown in Fig. 6). The electrodes 11.r, 11.c are configured for contacting a sample S. As illustrated in Fig. 6, at the crossings of the column electrodes 11.c and the row electrodes 11.r, an array of capacitances C0, C1, ... includes an electrical double layer capacitance C_EDL when the electrodes 11.r, 11.c are brought into contact with the sample S. The microelectrode device 1 includes an electronic equipment 12 which is configured for transmission of transmit signals V_TX0, V_TX1, ... to the row electrodes 11.r, and for reception of receive signals V_RX0, V_RX1, ... from the column electrodes 11.c in order to evaluate each capacitance of the array of capacitances C0, C1, ....

Fig. 7 schematically shows a signal processing method which enables evaluating each capacitance of the array of electrodes C0, C1, ... shown in Fig. 6. The electronic equipment is configured to apply a 2-D code division multiplexing. Each electrode of the row electrodes is driven by a synchronous and orthogonal set of transmit signals V_TX0, V_TX1, ... of orthogonal codes, such as pseudo-random Walsh codes. The capacitance C0, C1, ... between each column and row, which each include a respective electrical double layer capacitance C_EDL, represents the ion concentration of the corresponding coordinate. Each receive signal V_RX0, V_RX1, ... represents a weighted sum of capacitances C0, C1, ... modulated with Walsh codes. The receive signals V_RX0, V_RX1, ... are demodulated by a matrix multiplier using the original Walsh code set. After demodulation, the output voltages V_OUT0, V_OUT1, ... (cf. Fig. 6) each represent the capacitances C0, C1, ... located at a respective column. Accordingly, an electronic equipment 12 for a N-channel microelectrode device 1 requires only 2√N interconnects and √N readout circuits, relaxing integration complexity and improving area efficiency while measuring all channels concurrently.

Fig. 6, 7 illustrate four row electrodes 11.r and four column electrodes 11.c (4x4 electrodes), and a 2-D code division multiplexing. The invention is not limited to a particular number of electrodes. For example, the electrode assembly 11 can include 16x16 electrodes, 32x32 electrodes, etc. For example, an microelectrode device 1 with an electrode assembly 11 having 32x32 electrodes achieves 1,024 independent recording channels. The invention is not limited to square arrays. The number of row electrodes 11.r does not have to be as the number of column electrodes 11.c.

The electronic equipment (12) can include clock generators, bias circuits for controlling transmit circuits for transmission of transmit signals V_TX0, V_TX1, and for controlling readout circuits for reception of receive signals V_RX0, V_RX1, .... The transmit circuits can include controller circuits, weighting circuits, and similar for generating the transmit signals V_TX0, V_TX1, .... The readout circuits can include switching circuits serially connected to an amplifier chain, a decoder and a multiplexer. The amplifier chain can include low noise amplifiers (LNA), variable gain amplifiers (VGA), an analog buffer, and similar. The decoder can include multiplier circuits, switch capacitor integrators, and similar.

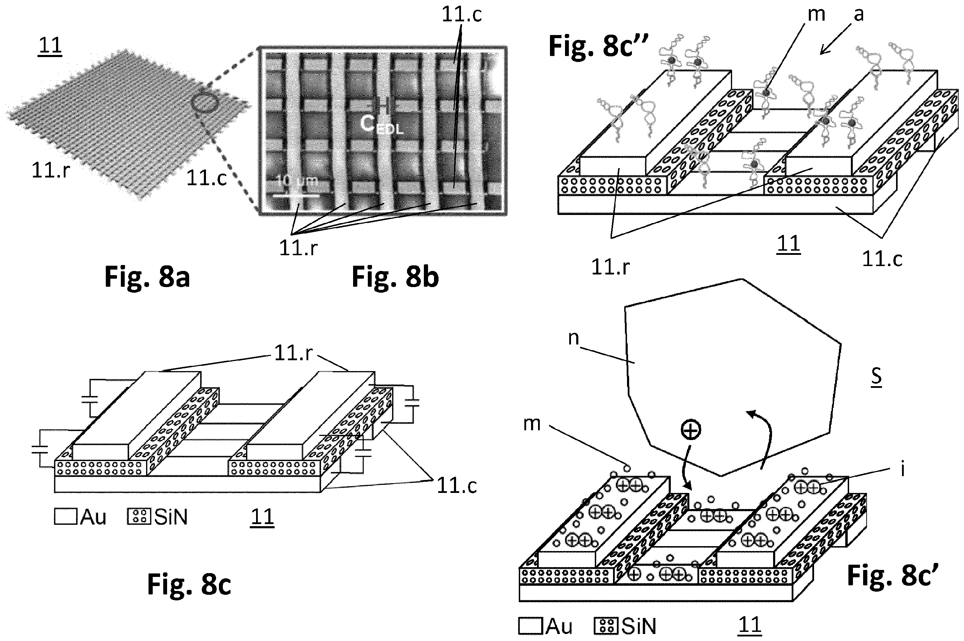

Fig. 8a shows an embodiment of an electrode assembly 11, which has a set of 32 row electrodes 11.r, and a set of 32 column electrodes. Fig. 8b shows a SEM image of a cutout of the electrode assembly 11. Fig. 8c schematically shows a 3-dimensional structure of a cutout of the electrode assembly 11 illustrated in Figs. 8a, 8b with electrodes 11.r, 11.c. Fig. 8c' schematically shows a 3-dimenstional structure of a cutout of the electrode assembly 11 illustrated in Figs. 8a, 8b with electrodes 11.r, 11.c for sensing an electrical activity of a neuron n. Fig. 8c" schematically shows a 3-dimenstional structure of a cutout of the electrode assembly 11 illustrated in Figs. 8a, 8b with electrodes 11.r, 11.c which are functionalized with aptamers a for sensing a chemical activity of molecules m.

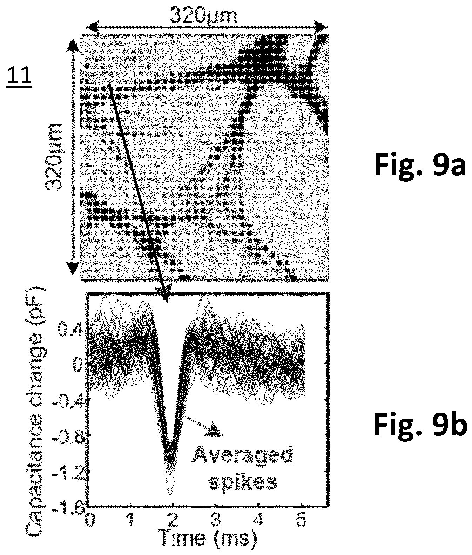

Fig. 9a shows a fluorescent image of an electrode assembly 11 having arranged a sample S with neurons. Fig. 9b shows for a particular location of the electrode assembly 11 the result of the electronic equipment 12 evaluating the capacitance at that location, wherein evaluation relates to determining a change of the capacitance.

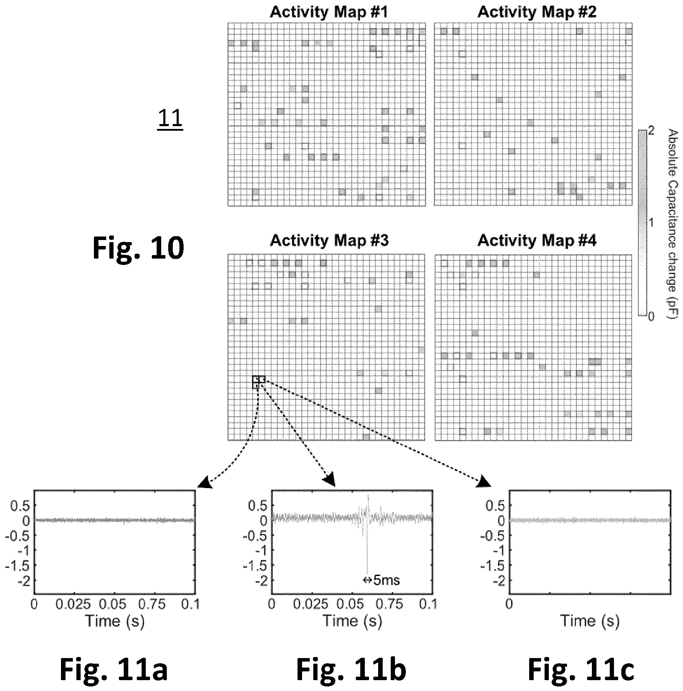

Fig. 10 shows a sequence of four activity maps of an electrode assembly 11 having arranged a sample S with neurons. The activity maps are generated by the electronic equipment 12 by evaluating the capacitances, wherein evaluation relates to determining a change of the capacitances.

Figs. 11a, 11b, 11c show the activity for activity map #3 at adjacent locations. Fig. 11b shows an activity at its location of the sample S, wherein Figs. 11a, 11c do not show an activity at their locations the sample S.

A distance between the electrode assembly 11 and the electronic equipment 12 can be 10 cm or more.

The electrode assembly 11 can have a transparent, soft or stretchable configuration such as based on PDMS (polydimethylsiloxane), hydrogels or similar.

The electrode assembly 11 can have a an corrode resistive configuration and can enable long-term exposure to liquids
The electrodes 11.1, 11.2; 11.r, 11.c of the electrode assembly 11 can include gold (Au), a conductive polymer such as PDOT:PSS (poly(3,4-ethylenedioxythiophene) polystyrene sulfonate), transparent conductors such as ITO (indium tin oxide), or similar.

The electrodes 11.1, 11.2; 11.r, 11.c of the electrode assembly 11 can be supported by a glass plate, a silicon substrate, or similar.

The electrodes 11.r, 11.c of the electrode assembly 11 can be fabricated by depositing two layers of gold (e.g. 150nm thickness) separated by a layer of SiN (silicon nitride) or another dielectric material such as PDMS, hydrogels, other nonconductive polymers or similar (e.g. 200nm thickness).

The electronic equipment 12 can be partially or fully fabricated in CMOS (Complementary Metal-Oxide-Semiconductor) or similar.

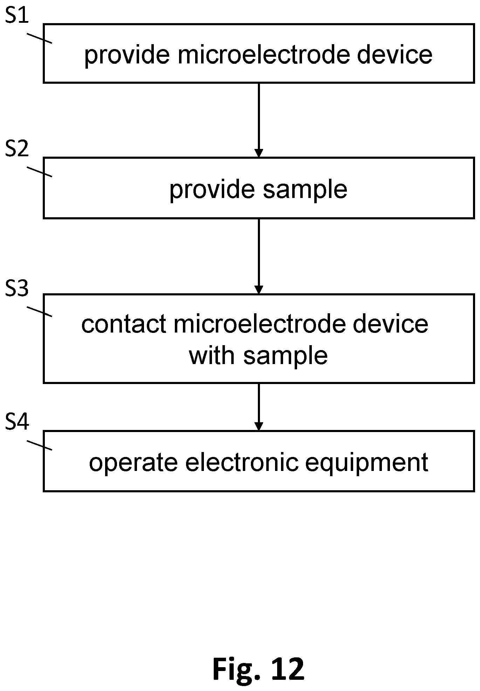

Fig. 12 shows schematically possible method steps of a method of using a microelectrode device 1 according to the present invention. In step S1, a microelectrode device 1 according to the present invention is provided. In step S2, a sample S is provided. In step S3, the sample S is brought in contact with the electrodes and forming within the sample S of an electrical double layer having an electrical double layer capacitance E_EDL at a surface of an electrode 11 is enabled. In step S4, the electronic equipment 12 is operated to evaluate a capacitance C for sensing the biological activity.

The method can further comprise a step of functionalizing the electrode assembly 11 for modifying the electrical double layer capacitance C_EDL depending on a chemical activity. For example, the electrode assembly 11 can be functionalized with aptamers a.

Functionalizing the electrodes with aptamers can include a preparation of aptamers.

In-Flow functionalization can be applied. The presence of a flow cell opens the possibility of a functionalization operated directly with the electronic equipment 12. With this approach, aptamers can be pushed with e.g. a syringe on the sensing area, allowing real-time monitoring of the changes in capacitance during functionalization, and an analysis of how much of this change is retained when rinsing with a buffer solution. An incubation time can be e.g. two hours.

Static functionalization can be applied. PDMS walls can be arranged to cleanly separate sides of the electrode assembly, so that solutions do not affect each other. Aptamer solutions can be applied with pipettes. An incubation time can be e.g. two hours. Different aptamer solutions can be applied.

Hybrid functionalization can be applied. First, an static functionalization can be applied, wherein a first set of aptamer solutions is used. Thereafter, within the setup, a further aptamer solution is applied for functionalizing remaining electrodes.

Functionalization using microcontact printing, chemical lift of lithography, or photochemical patterning, among others, are additional methods that can be used to functionalize aptamers to electrodes.

Functionalization of embodiments of a microelectrode array illustrated in Figs. 6, 8a, 8b, 8c can relate to pattern aptamers in the region where rows and columns overlap (cf. Fig. 8c"). Differential functionalization either between individual Rx, Tx, or electrodes is possible.

Finally, it should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

### LIST OF REFERENCE SIGNS

- 1: microelectrode device
- 11: electrode assembly
- 11.1, 11.2; 11.r, 11.c: electrodes
- 12: electronic equipment
- i: ion
- m: molecule
- n: neuron
- a: aptamer
- C_EDL: electrical double layer capacitance
- R_F: fluid resistor
- R_E: electrode resistor
- C; C0, C1,: capacitance between electrodes
- V_TX: transmit signal
- V_RX: receive signal

## Claims

1. A microelectrode device (1) for sensing a biological activity within a sample (S), the microelectrode device (1) comprising:
an electrode assembly (11) with electrodes (11.1, 11.2; 11.r, 11.c) for contacting the sample (S), and
an electronic equipment (12) which is connected to the electrodes (11.1, 11.2; 11.r, 11.c) of the electrode assembly (11),
wherein the electronic equipment (12) is configured to evaluate one or more capacitances (C; C0, C1, ...) between the electrodes (11.1, 11.2; 11.r, 11.c) for sensing the biological activity within the sample (S),
wherein the microelectrode device (1) enables that the one or more capacitances (C; C0, C1, ...) include one or more electrical double layer capacitances (C_EDL) of one or more electrical double layers which are formed when the electrodes (11.1, 11.2; 11.r, 11.c) are brought into contact with the sample (S).

2. The microelectrode device (1) according to claim 1, wherein the biological activity relates to an electrical activity within the sample (S), and wherein the electronic equipment (12) is configured to evaluate the one or more capacitances (C; C0, C1, ...) for sensing the electrical activity.

3. The microelectrode device (1) according to claim 1 or 2, wherein the biological activity relates to a chemical activity within the sample (S), and wherein the electrode assembly (11) is functionalized, in particular with aptamers i(a), for modifying the electrical double layer capacitance (C_EDL) depending on the chemical activity, and wherein the electronic equipment (12) is configured to evaluate the one or more capacitances (C; C0, C1, ...) for sensing the chemical activity.

4. The microelectrode device (1) according to one of claims 1 to 3, wherein the electrode assembly (11) includes electrodes which are arranged in a form of an array.

5. The microelectrode device (1) according to one of claims 1 to 4, wherein the electrode assembly (11) includes electrodes (11.r, 11.c) which form a cross-coupled capacitive microelectrode array, and wherein the electronic equipment (12) is configured to apply a code division multiplexing (CDM) method for evaluating the one or more capacitances (C0, C1, ...).

6. The microelectrode device (1) according to one of claims 1 to 5, wherein the electronic equipment (12) is configured to enable sensing of the biological activity with a frequency of 2 kHz or more.

7. The microelectrode device (1) according to one of claims 1 to 6, wherein the electrode assembly (11) is configured to enable sensing of the biological activity with a pitch of 50 micrometers or less.

8. The microelectrode device (1) according to one of claims 1 to 7, wherein the electrode assembly (11) has a transparent configuration, in particular enabling the use of standard microscopy equipment.

9. A method of using a microelectrode device (1) according to one of claims 1 to 9, comprising:
providing the microelectrode device (1),
providing a sample (S),
bringing the sample (S) in contact with the electrodes (11.1, 11.2; 11.r, 11.c) of the electrode assembly (11), and enabling forming within the sample (S) of one or more electrical double layers having one or more electrical double layer capacitances (C_EDL) at one or more surfaces of the electrode (11),
operating the electronic equipment (12) to evaluate the one or more capacitances (C; C0, C1, ...) for sensing the biological activity.

10. The method according to claim 9, wherein the biological activity relates to an electrical activity, and wherein the electronic equipment (12) is operated for sensing the electrical activity.

11. The method according to claim 9 or 10, wherein the biological activity relates to a chemical activity, and wherein the method further comprises functionalizing the electrode assembly (11), in particular with aptamers (a), for modifying the electrical double layer capacitance (C_EDL) depending on the chemical activity, and wherein the electronic equipment (12) is operated for sensing the chemical activity.

12. The method according to claim 11, wherein functionalizing the electrode assembly (11) includes one or more of: an in-flow functionalization, an outside functionalization, a hybrid functionalization, and a microfluidics functionalization.

13. The method according to one of claims 9 to 12, wherein the sample (S) includes one or more of electrogenic cells, chemogenic cells and cells that have ion channel activities.

14. The method according to one of claims 9 to 13, wherein the sample (S) includes one or more of neurons, glia, muscle cells, and cardiomyocites.

15. The method according to one of claims 9 to 14, wherein the sample (S) is provided in-vitro or in-vivo.
